(19) Europäisches Patentamt
European Patent Office
Office européen des brevets

(11) Veröffentlichungsnummer : **0 221 969 B1**

(12) # EUROPÄISCHE PATENTSCHRIFT

(45) Veröffentlichungstag der Patentschrift : **31.07.91 Patentblatt 91/31**

(51) Int. Cl.$^5$ : **A61B 5/00**, A61B 5/0448

(21) Anmeldenummer : **86903216.9**

(22) Anmeldetag : **17.05.86**

(86) Internationale Anmeldenummer : **PCT/DE86/00210**

(87) Internationale Veröffentlichungsnummer : **WO 86/06945 04.12.86 Gazette 86/26**

(54) **EINSTICHELEKTRODENANORDNUNG ZUR KONTINUIERLICHEN pO 2?-MESSUNG IM LEBENDEN HAUTGEWEBE.**

(30) Priorität : **23.05.85 DE 3518463**

(43) Veröffentlichungstag der Anmeldung : **20.05.87 Patentblatt 87/21**

(45) Bekanntmachung des Hinweises auf die Patenterteilung : **31.07.91 Patentblatt 91/31**

(84) Benannte Vertragsstaaten : **AT BE CH FR GB IT LI LU NL SE**

(56) Entgegenhaltungen :
**EP-A- 0 104 493**
**DE-A- 2 930 663**
**FR-A- 2 120 787**
**US-A- 3 415 730**

(56) Entgegenhaltungen :
**US-A- 4 294 258**
**Medical Instrumentation, Appl. & Design, J. Webster, 1978, Houghton Mifflin Comp., Seite 253**

(73) Patentinhaber : **SCHUHMANN, Rolf, Prof. Dr. med.**
**Gravenbrucher Weg 19**
**W-6056 Heusenstamm (DE)**

(72) Erfinder : **SCHUHMANN, Rolf, Prof. Dr. med.**
**Gravenbrucher Weg 19**
**W-6056 Heusenstamm (DE)**

(74) Vertreter : **Meier, Robert, Dipl.-Ing.**
**Patentanwalt Dipl.-Ing. Robert Meier Auf dem Mühlberg 16**
**W-6000 Frankfurt am Main 70 (DE)**

**EP 0 221 969 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung bezieht sich auf eine Anordnung mit einer Einstichelektrodenanordnung zur kontinuierlichen $pO_2$ -Messung und Messung der fösalen Herzfrequenz im lebenden und unruhig bewegten Gewebe, beispielsweise in der Haut des vorangehenden Teiles eines Kindes während der Geburt.

Ein Verfahren und eine Elektrodenanordnung zur transkutanen $pO_2$-Messung sind von LÜBBERS und HUCH (1960) entwickelt worden. Während dieses Verfahren in der Intensivpflege von Neugeborenen sich durchaus bewährt hat, ist seine Anwendung, beispielsweise im Bereich der Geburtshilfe, problematisch : Einmal ist die Anbringung der von LÜBBERS und HUCH vorgesehenen Elektroden (Außendurchmesser 18 mm) nicht einfach, zum anderen werden diese Elektroden mit 44° C beheizt, so daß zwar der "arterielle" Sauerstoffpartialdruck gemessen werden kann, die Heizung andererseits aber nach etwa 3 Stunden Hautveränderungen verursachen kann, mit der Folge,daß die Elektrode an einer anderen Stelle angebracht werden muß.

Eine $pCO_2$-Elektrodenanordnung (LÜBBERS, HUCH, 1980) mißt ebenfalls transkutan ; für dieses Meßsystem gilt dasselbe, wie für die transkutane $pO_2$-Elektrodenanordnung.

Die Messung des Sauerstoffpartialdruckes beruht auf dem Phänomen der Polarisation, d.h., Sauerstoffmoleküle, welche an eine Platinkathode gelangen, erzeugen einen elektrischen Strom, welcher der Anzahl der Sauerstoffmoleküle proportional ist. Die Elektrode liegt an einer Polarisationsspannung von beispielsweise -780 mV und ist so aufgebaut, daß der Sauerstoff aus dem Blut bzw, dem Gewebe durch eine dünne, elektrisch isolierende, aber gasdurchlässige Membran zur Platinkathode diffundiert. Der durch die Elektrode fließende Strom (Polarisationsstrom) wird mittels eines Amperemeters gemessen. Er ist dem Sauerstoffpartialdruck im Gewebe an der Elektrodenspitze proportional. Da aber keine absolute Reaktion zwischem dem Polarisationsstrom und dem an der Elektrodenspitze herrschenden $pO_2$ besteht, muß jede Sauerstoffelektrode mit Eichgasen, deren $pO_2$ bekannt ist, geeicht werden (0,9 % NaCL-Lösung mit dem bekannten Sauerstoffgehalt - 20 %, 10 %, 0 % - [0,9 % - NaCL-Lösung mit Stickstoff anteilig äquilibriert]).

Die allgemeine Reaktion ist :

$$O_2 + 4\ H^+ + 4\ e^- \longrightarrow 2\ H_2O \text{ Kathode (Meßelektrode)}$$
$$4\ Ag + 4\ CL^- \longrightarrow 4\ AgCL + 4\ e^- \text{ (Anode)}$$

Gesamtreaktion

$$O_2 + 4\ Ag + 4\ H^+ + 4\ CL^- \longrightarrow 4\ AgCL + 2\ H_2O$$

Im Jahre 1956 führte CLARK eine Platinelektrode zur Messung des Sauerstoffpartialdruckes ein. Seit dieser Zeit sind eine Reihe von $pO_2$-Elektrodenanordnungen nach CLARK entwickelt worden. Mikround Makroelektroden als gerade Stichelektroden für $pO_2$-Messungen im menschlichen Gewebe sind in zunehmendem Maße in der Chirurgie, der inneren Medizin und Pharmakologie zum Einsatz gekommen (erstes Symposium der Gewebesauerstoff-Druckmessungen 1982, zweites Gewebesauerstoffdruck Symposium 1983 in Frankfurt "Determination of Tissue Oxigen Pressure in Patient", Professor A.M. Ehrly, Pergamon-Press).

Stichelektroden mit gerader Form für $pO_2$-Messungen haben sich überall da bewährt, wo das zu untersuchende Gewebe des patienen bzw. des Kindes relativ ruhig liegen.

Bekanntgeworden ist beispielsweise eine Einstichelektrode zur kontinuierlichen $pO_2$-Messung im lebenden Hautgewebe, bestehend aus einer Kathode, die gasdicht in die Glaskapillare eingeschmolzen und in einem Hohlkörper fixiert ist, wobei der Hohlkörper über ein Netz mit der Abschirmung des Koaxialkabels derart verbunden ist, daß das Netz die Harzeinbettung, die Verbindungsstelle der Kathode mit der Kupferseele des Koaxialkabels vollständig umgibt, rundum bedeckt, und bei der ein Rohr, vorzugsweise bestehend aus Silber, kanülartig eingeschliffen ist und am anderen Ende eine Verdickung aufweist, wobei ein Haken fest mit dem Rohr verbunden ist und an der unteren Seite des Elektrodenkörpers eine Auflagefläche, die unter dem vorderen Hohlraum des Hakens eine beiderseitige Aussparung besitzt, aufweist (DD88 835).

Das besondere an dieser bekannten Einstichelektrodenanordnung zur kontinuierlichen $pO_2$-Messung im lebenden Hautgewebe ist darin zu sehen, daß die äußere, als Anode dienende Metallrohrelektrode aus Silber/Silberoxid besteht und als Referenzelektrode dient. Die darin elektrisch isoliert angeordnete Platindrahtelektrode dient als eigentliche Meßelektrode mit einem Durchmesser von annähernd 50 μm.

Mit einer solchen Elektrodenanordnung lassen sich exakte Messungen nur im ruhenden Gewebe durchführen. Zum Festsetzen ist die Einstichelektrodenanordnung mit einem Haken versehen, welcher während des

Einsatzes mit einer chirurgischen Klammer festgelegt wird.

Diese Elektrodenanordnungen sind jedoch nicht anwendbar, wodie Sauerstoffpartialdruckmessungen an einem beweglichen Patienten, vorzugsweise bei einem Kind unter der Geburt vorgenommen werden müssen.

Aber auch andere bekannt gewordene Elektrodenanordnungen sind nicht imstande, eigenen sich nicht zu Messungen des Sauerstoffpartialdruckes in der Haut eines sich bewegenden Patienten.

Gemäß US-A- 3 415 730 ist eine Elektrodenanordnung mit einer geraden Elektrodennadel, mit der eine pH-Wert-Messung direkt im Blutstrom (nicht im Gewebe) vorgenommen werden soll. Es wird kein Weg aufgezeigt, wie die gerade Elektrodennadel unter der Geburt in die Haut des Kindes appliziert und gehalten oder in eine spiralige Form gebracht werden konnte, damit sie unter der Geburt sicher in der Haut des Kindes fixiert bleibt.

Elektrodenanordnungen nach dem US-A- 4 294 258 eignen sich nur zur Messung des pH-Wertes. Nicht aber zur $pO_2$ -Messung, weil dabei die Meßelektrode als Meßsensor bis an die Meßstelle geführt werden muß, was mit den bekannten Elektrodenanordnungen nicht möglich ist. Diese sind zudem sog. Makro-Elektrodenanordnungen, die sich ohne Schäden zu verusachen nicht innerhalb der Gebärmutter oder des Geburtskanals in der Haut des Kindes applizieren lassen.

Auch eine gerade Elektrode nach EP-A- 0 104 493 kann nicht zu einer Spiralanordnung gebogen werden und eignet sich nicht zu $pO_2$-Messungen in der Haut eines Kindes unter der Geburt.

Daher liegt der vorliegenden Erfindung die Aufgabe zugrunde, eine Anordnung mit einer Einstichelektrodenanordnung zum Messen des $pO_2$-Partialdruckes an unruhigen und beweglichen Geweben zu schaffen, mit der zugleich auch, unabhängig von der Messung des $pO_2$-Partialdruckes, andere Messungen vorgenommen werden können.

Diese Aufgabe wird erfindungsgemäß durch eine Anordnung mit einer Einstichelektrodenanordnung nach den Ansprüchen 1 und 10 gelöst.

Hierdurch ist es möglich, kontinuierliche $pO_2$ -Messungen unter der Geburt durchzuführen. Die Elektrodenanodnung wird einfach in die Haut des kindlichen Gewebes, und zwar des Gewebes des vorangehenden Teiles, eingedreht. Die Elektrodenanordnung sitzt - wie Versuche ergeben haben - so fest im Gewebe, daß die $pO_2$-Meßwerte und die Herzfrequenz-Meßwerte zuverlässig wärend der gesamten Geburt zur Verfügung stehen. Als Bezugselektroden werden Ag/Ag - CL-Elektroden unabhängig voneinander an der Hautoberfläche der Mutter fixiert. Mit Hilfe der neuartigen Anordnung mit einer Einstichelektrodenanordnung ist es demnach möglich, unabhängig voneinander sowohl den $pO_2$-Druck wie auch die Herzfrequenz unter der Geburt des Kindes zu messen. Die unabhängig voneinander ermittelten Meßergebnisse lassen sich durch elektronische Auswertgeräte so miteinander in Beziehung setzen, daß der Arzt unter der Geburt verlässliche Aussagen über den $pO_2$-Druck und die Herzfrequenz des Kindes erhält.

Die Messung dieser beiden sehr wichtigen Aussagen mit nur einer Einstichelektrodenanordnung war bisher nicht möglich.

Zwar sind zum Festlegen im Gewebe spiralige Elektroden bekannt. (EP 0 104 619, US-PS 4 320 764), jedoch eignen sich diese nicht zur Lösung der Erfindungsaufgabe. Die EP 0 104 619 bezieht sich auf die Kombination einer spiraligen EKG-Elektrode mit einer Einrichtung zur Messung des pH-Wertes. Hierzu werden Lichtleiter verwendet, die in eine spiralige Hohlnadel eingeschoben werden. Die aus dem spiraligen Schutzrohr herausgeführten Enden der Lichtleiter sind an eine Lichtquelle bzw. an einen Lichtsensor angeschlossen.

Der Durchmesser der spiraligen Schutzrohrelektrode ist so groß, daß die beiden Lichtleiter ohne Schwierigkeiten darin Platz finden. Die Lichtleiter brauchen nicht untereinander und auch nicht gegenüber der metallischen Schutzrohrelektrode isoliert zu werden.

Die Messung des pH-Wertes ist unter Umständen zwar von Bedeutung, sie kann jedoch die Messung des $pO_2$-Druckes nicht ersetzen.Durch die bekannte Elektrodenanordnung mit Lichtleitern konnten keine Anregungen gegeben werden, zwei Metallelektroden elektrisch voneinander isoliert ineinander angeordnet spiralig auszubilden und jede der Metallelektroden an ein Meßgerät mit einer eigenen Bezugselektrode anzuschließen, so daß unabhängig voneinander unter der Geburt zwei wichtige Meßreihen, nämlich über den $pO_2$-Druck und über die Herzfrequenz erzielbar werden.

Die US-PS 4 320 764 bezieht sich auf eine spiralige Elektrode, die mit ihrem Anschlußende in einem Kunststoffkörper sitzt. An dem der spiraligen Elektrode gegenüberliegenden Ende des Kunststoffkörpers ist eine metallische Elektrode angebracht. Zwischen den beiden Elektroden bildet sich infolge ihrer chemischen Beschaffenheit und des Fruchtwassers ein elektrisches Potential aus, welches Aufschluß über die jeweiligen pH-Werte des Fötus gibt.

Eine gleichzeitige Messung zweier wichtiger Meßreihen wie mit der Anordnung nach der Erfindung, ist nicht möglich.

Auch mit einer spiraligen Nadelelektrode aus nichtrostendem Stahl gemäß : "Medical instrumentation Application and design" J. Webster 1978, Houghton Mifflin Comp. Seite 253 ist die Lösung der Erfindungsaufgabe nicht möglich, da es sich hierbei nicht um ein spiralige Hohlnadel handelt, in der sich eine isolierte spiralige Platindrahtelektrode befindet.

Um eine sichere Isolation zwischen den Metallelektroden nach der Erfindung zu ermöglichen, ist die Platindrahtelektrode innerhalb der Metallrohrelektrode von einer Cellophanhülse umgeben. Hierdurch wird im wesentlichen verhindert, daß schon während des Herstellungsvorganges der Elektrodenanordnung die Isolation zwischen der Platindrahtelektrode und der Metallrohrelektrode beeinträchtigt wird.

Um ganz sicher zu gehen, ist zusätzlich der Innenraum der Metallrohrelektrode mit Kunstharz, ARALDIT XW 39 Ciba-Geigy, BRD, vorzugsweise unter Anwendung von Vakuum, ausgefüllt.

Einzelheiten der Elektrodenanordnung sind in den Ansprüchen 4 und 5 gekennzeichnet.

Besonders wichtig ist, daß die Metallrohrelektrode mit dem Anschlußende der Platindrahtelektrode außermittig im Randbereich der vorderen Fläche des Kunststoffkörpers sitzt. Hierdurch wird erreicht, daß die als offene Spirale ausgebildete Elektrodenanordnung vor der vorderen Fläche des Kunststoffkörpers angeordnet sein kann, wobei der Durchmesser dieser spiraligen Elektrodenanordnung kleiner ist als der Durchmesser des Kunststoffkörpers.

Im Mantel des Kunststoffkörpers sind zwei einander gegenüberliegende Fixierstücke angeordnet, die aus der Mantelfläche herausragen. Sie dienen in Verbindung mit einer Einführhilfe dazu, die Elektrodenanordnung - vorzugsweise unter der Geburt - in das Gewebe des vorangehenden Teiles des Kindes einzudrehen.

Einzelheiten der Anordnung ergeben sich aus den Unteransprüchen.

Gemäß ansprüche 1 und 10 ist die vordere Endfläche der Elektrodenanordnung eine Abschliff-Fläche mit einem Neigungswinkel $\alpha$ von etwa 20° gegenüber einer durch den vorderen Bereich der Metallrohrelektrode sich erstreckenden Querschnitts-fläche. Hierdurch wird das Eindrehen der Elektrodenanordnung in das Gewebe der Haut erleichtert. Dabei kann die Meßfläche der Platindrahtelektrode blank auf das Gewebe treffen. Hierbei tritt über der Meßfläche (Platin) eine kleine $pO_2$-Verarmungszone auf, die meßtechnisch zwar sehr klein ist, jedoch keine stabilen Verhältnisse gewährleistet. Infolge dessen hat eine derartige Meßelektrode zwar eine hohe Ansprechempfinglichkeit, sie ist jedoch außerordentlich bewegungsempfindlich bzw. rührempfindlich. Solche Elektrodenanordnungen können mit Vorteil immer da eingesetzt werden, wo es im wesentlichen nur auf eine Anspechempfindlichkeit ankommt. Exakte Meßwerte werden kaum oder nur dann geliefert, wenn die Störfrequenzen elektronisch ausgeblendet werden.

Eine Verbesserung der Meßergebnisse läßt sich dann erzielen,wenn die Abschliff-Fläche und die Meßfläche mit einer elektrisch isolierenden, gasdurchlässigen Membran überzogen sind. In Abhängigkeit von der Dicke dieser Membran wird die $pO_2$-Verarmungszone flacher, d.h. die Ansprechempfindlichkeit sowie der Rühreffekt geringer. Durch die Dicke der Membran kann man die entsprechenden Werte einstellen bzw. regulieren.

Im vorliegenden Fall ist das Überziehen der Anschliff-Fläche mit einer Membran jedoch schwierig, weil sich der vordere Bereich der Elektrodenanordnung von außen her aus einem Metall-($V_2A$) Mantel, einem Kunstharzmantel, einem Cellophanmantel, einem darin liegenden Kunstharzmantel und der Platindrahtmeßelektrode aufbaut. Es kann während der Benutzung der Elektrodenanordnung in Flüssigkeit zu "Abhebungserscheinungen" der Membran kommen mit der Folge, daß die Elektrodenanordnung nach jedem Gebrauch neu beschichtet werden muß. Durch die neue Beschichtung trat eine Änderung der Meßwerte auf, so daß die Elektrodenanordnung neu geeicht werden mußte. Versuche haben ergeben, daß sich eine Verringerung der Verarmungszone vor der Meßfläche durch eine sog. Recessionsstrecke erreichen läßt, um die die Meßfläche der Meßelektrode hinter der Abschliff-Fläche zurückversetzt ist.

Besonders gute Ergebnisse werden mit Elektrodenanordnungen erzielt, deren vorderer Bereich gem. Anspruch 10 eine Recessionsstrecke aufweist, die mit einer Membran überzogen ist, deren rückwärtiger Teil die Recessionsstrecke ausfüllt. Hierdurch erreicht man eine definierte Dicke der Membran. Hinzu kommt, daß die Membran an der Kunstharzwand innerhalb des Schutzrohres besser haftet und somit nicht zu Ablösungserscheinungen neigt. Bei Tierversuchen sind Elektroden dieser Bauart teilweise über 48 Stunden im Gewebe geblieben, ohne daß hinsichtlich der Meßwerte Drifts oder Änderungen festgestellt werden konnten. Die Eichkurve vor dem Versuch änderte sich gegenüber derjenigen nach dem Versuch um weniger als 5 %.

Ein Verfahren zum Herstellen einer derartigen Elektrodenanordnung bestehend aus einer Platindrahtelektrode und einer umgebenden elektrisch davon isolierten Metallrohrelektrode, vorzugsweise aus V A-Stahl, mit einer Recessionsstrecke im vorderen Bereich, wird in Anspruch 14 definiert.

Im einzelnen geht dieser Abbau so vor sich, daß der vordere Bereich der Elektrodenanordnung in eine Salzlösung eingegeben wird, in der sich ein Platin-Plättchen befindet, woraufhin für die Dauer einer der gewünschten Länge der Recessionsstrecke entsprechend einstellbaren Zeitspanne die Platindrahtelektrode an den Minuspol und das Platin-Plättchen an den Pluspol einer Gleichspannungsquelle angeschlossen werden und daß anschließend eine gründliche Säuberung des vorderen Bereiches der Elektrodenanordnung erfolgt.

Weitere Einzelheiten dieses Verfahrens ergeben sich aus den Ansprüchen 16 und 17.

Nach der Säuberung wird dann die Beschichtung des vorderen Bereiches der Elektrodenanordnung mit

der Membran und das Auffüllen der Recessionsstrecke mit dem Material der Membran unter Vakuumbedingungen vorgenommen.

Ausführungsbeispiele der Erfindung werden nachfolgend anhand der Zeichnung erläutert.

Es zeigt :

Fig. 1 eine perspektivische, vergrößerte Ansicht der Elektrodenanordnung,

Fig. 2 eine Seitenansicht der Elektrodenanordnung gemäß Fig. 1,

Fig. 3 Schnitte durch verschiedene Ausbildungsformen des vorderen Bereiches der Elektrodenanordnung,

Fig. 4 einen Schnitt durch eine Einführhilfe mit der Elektrodenanordnung,

Fig. 5 schematisch die Darstellung der Applizierung der Elektrodenanordnung am vorangehenden Teil eines Kindes unter der Geburt,

Fig. 6 eine stark vereinfachte Darstellung der $pO_2$-Verteilung im oberen Bereich der Haut, eine grafische Darstellung und schematisch eine Darstellung der Funktionsweise einer Anwendung der Erfindung und

Fig. 7 eine schematische Darstellung der Verwendung der erfindungsgemäßen Elektrodenanordnung.

Fig. 1 zeigt in perspektivischer Ansicht eine Elektrodenanordnung 1 mit zwei spiralig ausgebildeten Metallelektroden 2, 14, die im wesentlichen aus einer Metallrohrelektrode 14 und einer darin elektrisch isoliert angeordneten Platindrahtelektrode 2 sowie einem zylindrischen Kunststoffkörper 26 besteht, der in einem bevorzugten Ausführungsbeispiel aus Araldit XW 39 (der Firma Ciba-Geigy) hergestellt ist. Gemäß Fig. 2 ist seine Höhe mit H und sein Durchmesser mit D bezeichnet. In einem bevorzugten Ausführungsbeispiel beträgt die Höhe 5 mm und der Durchmesser 4,5 mm.

Aus einer vorderen Fläche 29 des Kunststoffkörpers 26 ragt außermittig, d.h. aus ihrem Randbereich die Metallrohrelektrode 14 heraus, in welcher die Platindrahtelektrode 2 angeordnet ist. Die Metallrohrelektrode 14 ist als sog. offene Spirale mit einer Steigung S (Fig. 2) von ca. 30° ausgebildet. Die Platindrahtelektrode 2 weist eine vordere Meßfläche 4 auf, die - wie weiter unten im einzelnen erläutert - Teil einer nach außen weisenden Abschliff-Fläche 23, 23a mit einem Neigungswinkel $\alpha$ gegenüber der Metallrohrelektrode 14 von etwa 20° ausgebildet ist.

Die als $pO_2$-Sensor ausgebildete Platindrahtelektrode 2 besitzt ein Anschlußende 6, welches über eine Verbindungsleitung 21 an eine Leitung 51 angeschlossen ist, welche gemäß Fig. 7 mit einem $pO_2$-Meßgerät 53 in Verbindung steht.

Wie vor allem die Fig. 3a - 3e erkennen lassen, sitzt die Platindrahtelektrode 2 elektrisch isoliert in der Metallrohrelektrode 14. Die Platindrahtelektrode 2 ist innerhalb der Metallrohrelektrode 14 von einer Cellophanhülse 7 umgeben. Zusätzlich ist der Innenraum der Metallrohrelektrode 14 mit Kunstharz 10 ausgefüllt. Um eine sehr sichere Isolation der Platindrahtelektrode gegenüber der Metallrohrelektrode zu erreichen, ist das Kunstharz 10 vorzugsweise unter Anwendung von Vakuum in den Innenraum der spiraligen Metallrohrelektrode 14 eingebracht.

Im einzelnen dient die Platindrahtelektrode 2 als $pO_2$-Sensor und weist einen Durchmesser d von etwa 20 µm auf. Sie ist in die Cellophanhülse 7 eingefädelt, welche ihrerseits einen Außendurchmesser 1 von etwa 200 µm und einen Innendurchmesser i von etwa 180 µm aufweist. Die Metallrohrelektrode 14, welche in bevorzugten Ausführungsbeispielen aus $V_2A$-Stahl besteht, besitzt einen Außendurchmesser ad von etwa 450 µm und einen Innendurchmesser id von etwa 300 µm. Diese Abmessungen sichern einerseits eine gute Gebrauchsfähigkeit der Elektrodenanordnung und darüber hinaus eine verhältnismäßig einfache Herstellung.

Aus dem Mantel 30 des Kunststoffkörpers 26 ragen, etwa 1 mm von der vorderen Fläche 29 entfernt, zwei einander gegenüberliegende Fixierstifte 31 heraus, deren Bedeutung weiter unten erläutert wird.

Fig. 1 läßt erkennen, daß an die Metalldrahtelektrode 14 elektrisch leitend eine Verbindungsleitung 22 angeschlossen ist, die zu einer Leitung 52 (Fig. 7) führt, welche im dargestellten Ausführungs-beispiel über einen Anschlußblock an ein Verbindungskabel 52a angeschlossen ist, das seinerseits mit einem weiteren Meßgerät CTG (Kardiotokograph zur analyse und Registrierung von wehentätighert und fösalen Herzfrequenz) in Schaltverbindung steht, durch welches unter der Geburt ein peripheres kardiales Aktionspotential zur Errechnung oder zur Bestimmung der Herzfrequenz und mittels einer Wehen aufnehmers (56) die Wehentätigheit abgeleitet wird.

Vor allem Fig. 1 läßt deutlich erkennen, daß die Metallrohrelektrode 14 mit der umschlossenen Platindrahtelektrode 2 eine (Teil-) Spiralwindung mit einer Steigung S von ca. 30° bildet, deren Außendurchmesser A kleiner ist als der Durchmesser D des Kunststoffkörpers 26.

Ein Ausführungsbeispiel der Elektrodenanordnung 1 kann wie folgt hergestellt werden :

I. <u>Kunststoffkörper 26</u>

Zunächst wird von einer gegossenen Kunstharzstange aus Araldit XW39C mit einem Außendurchmesser D von 4,5 mm eine Scheibe mit einer Dicke von 3 mm abgeschnitten. Von der Schnittfläche her wird ca. 0,5

mm vom Mantel 30 entfernt ein Loch mit einem Durchmesser von 0,5 mm für das Anschlußende 6 der Metallrohrelektrode 14 und für die Verbindungsleitung 21 gebohrt. Außerdem wird quer durch die Kunstharzstange, 2 mm von der Schnittfläche entfernt, ein Loch mit einem Durchmesser von 0,3 mm zur Aufnahme der Fixierstifte 31 gebohrt.

II. Platindrahtelektrode 2

Ein Platindraht von 0,02 mm Durchmesser d und einer Länge von 5 cm wird in eine Cellophanhülse 7 von 3 cm Länge mit einem Außendurchmesser a von 0,2 mm und einem Innendurchmesser i von 0,18 mm eingefädelt. Zur Stabilisierung wird in das eine Ende der Cellophanhülse 7 ein 0,07 mm dicker Platindraht zusätzlich eingeführt. Dieser reicht nur etwa 3 mm weit in die Cellophanhülse 7 hinein und dient zur Stabilisierung des 0,02 mm dicken Platindrahtes bei der späteren Verlötung. Die Cellophanhülse 7 wird daraufhin in die noch gerade Metallrohrelektrode 14 gebracht, so daß ihr Ende mit den zweï Platindrähten 1 mm weit aus dem Metallrohr herausragt. Danach wird das Metallrohr 2 mm vom Ende, aus dem die beiden Platindrähte herausragen, um einen Winkel von etwa 30° gebogen und dieses abgebogene Ende mit einer Rundzange (4 mm Ø) um 180° gewendelt. Anschließend wird die die Platindrahtelektrode 2 enthaltene Metallrohrelektrode 14 in flüssiges Kunstkarz gelegt, und das Gefäß für 10 Minuten einem Vakuum von $10^{-4}$ Torr ausgesetzt. Hierdurch wird bei der Aufhebung des Vakuums gewährleistet, daß das Kunstharz alle Hohlräume in der Metallrohrelektrode 14 ausfüllt. Danach wird die gewendelte Elektrodenanordnung 1 für 24 Stunden getrocknet. Anschließend wird der vordere Bereich 17 der Metallrohrelektrode 14 und der Platindrahtelektrode 2 in einem Winkel von 20° mit Naßschleifpapier (Nr. 400) geschliffen und poliert, wodurch die Anschleiffläche 23 entsteht. Durch Spezialschliff kann auch eine linsenförmig zurückgesetzte Abschliff-Fläche 23a erzeugt werden.

Die Metallrohrelektrode 14 wird nun in die obere seitliche Bohrung in der vorderen Fläche 29 des Kunststoffkörpers 26 eingeführt und verklebt. Die beiden Platindrähte ragen danach aus dem gegenüberliegenden Ende der Bohrung heraus. zur Verlötung hat sich bewährt, ein Kupferplättchen als Fixpunkt für die Litze einzusetzen. Hierzu wird ein Kupferblech von 2 mm Durchmesser (Dicke 0,05 mm) gegenüber der Bohrung mit den beiden herausragenden Platindrähten am Elektrodenkörper verklebt. Mit diesem Kupferplättchen werden nun die herausragenden Enden der beiden Platindrähte verlötet. Danach wird der Kunststoffkörper 26 in einen PVC-Schlauch (Innendurchmesser 4,5 mm) eingeführt, so daß die Metallrohrelektrode 14 mit dem Ende des PVC-Schlauches abschließt. Die Länge des PVC-Schlauches beträgt 5 mm. Die Kupferlötstelle wird nun mit einer 0,4 mm starken Kupferlitze verlötet. Der Raum zwischen dieser Lötstelle im Inneren und der Oberkante des PVC-Schlauches wird mit Kunstharz ausgegossen. Nach 24 Stunden Trocknung wird der PVC-Schlauch entfernt.

Nun wird durch die Querbohrung im Kunststoffkörper 26 ein Stahlstift geführt und verklebt, so daß seine Enden rechts und links ca. 1 mm weit über den Mantel 30 herausragen und die Fixierstifte 31 bilden.

Im einfachsten Fall wird der vordere Bereich 17 der Metallrohrelektrode 14 mit der Meßfläche 4 der Platindrahtelektrode 2 mit Polystrol (Kunststoff-Handbuch, Bd. I., Grundlagen, S. 936, Carl Hauser Verlag) verschiedener Konzentrationen bezogen (getaucht) und getrocknet. Dieser Überzug dient ais Membran 12 bzw. 12a. Anschließend wird die Elektrodenanordnung 1 in einer $0_2$ äquilibrierten und $N_2$ gesättigten 0,9 %igen NaCL-Lösung ausgemessen bzw. geeicht.

III. Vor Einführung der Metallrohrelektrode 14 in den Kunststoffkörper 26 wird an ihren geraden Teil eine Kupferlitze (Außendurchmesser 0,4 mm) außen angebracht und durch die Bohrung mit den Platindrähten geführt und verklebt. Der weitere Verlauf ist sonst wie oben beschrieben.

Die Fig. 3a - 3e zeigen verschiedene Ausbildungsformen des vorderen Bereiches 17 einer Elektrodenanordnung 1. Hierbei spielt es keine Rolle, ob die Metallrohrelektrode 14 gewendelt ist oder zu einer sog. Nadelelektrode gehört.

Fig.3a zeigt einen vergrößerten Querschnitt durch den vorderen Bereich 17 einer Elektrodenanordnung 1. Innerhalb der Metallrohrelektrode 14 ist die Platindrahtelektrode 2 angeordnet. Gewöhnlich besteht die Metallrohrelektrode 14 aus $V_2A$-Stahl.

Die Platindrahtelektrode 2 endet in der Meßfläche 4. Um die Platindrahtelektrode 2 herum ist die Cellophanhülse 7 angedeutet. Die Darstellungen der Fig. 3a - 3e sind im vergrößerten Maßstab gezeichnet, wobei - der besseren Klarheit wegen - die einzelnen Abstände und Dicken der Bestandteile der Elektrodenanordnung 1 nicht in den natürlichen Proportionen dargestellt sind.

Die Metallrohrelektrode 14 und die Cellophanhülse 7 sind mit Kunstharz 10 ausgefüllt.

Das Ende des vorderen Bereiches 17 der Elektrodenanordnung 1 weist eine Abschliff-Fläche 23 auf, die gegenüber der Längswand des Schutzrohres 14 um einen Neigungswinkel $\alpha$ von etwa 20° gegenüber einer durch den vorderen Bereich 17 der Metallrohrelektrode 14 sich erstreckenden Querschnittsfläche geneigt ist.

Ein vorderer Bereich 17 gemäß Fig. 3a bildet sich während des Betriebes oberhalb der Meßfläche 4 eine

sog. kleine $pO_2$-Verarmungszone 24 aus, die meßtechnisch zwar sehr klein ist, jedoch keine stabilen Meßergebnisse liefert. Eine Elektrodenanordnung gemäß Fig. 3a weist zwar eine hohe Ansprechempfindlichkeit auf, hat andererseits jedoch eine große Bewegungsempfindlichkeit, vorzugsweise Rührempfindlichkeit. Für direkte, exakte Messungen sind derartige Elektroden wenig geeignet. Die Meßwerte unterliegen großen Schwankungen und können lediglich durch Mittelwertbildung durch Computer o.dgl. einer sinnvollen Auswertung zugeführt werden.

Wenn gemäß Fig. 3b die Abschliff-Fläche 23 jedoch mit einer Membran 12 überzogen wird, wird die $pO_2$-Verarmungszone in Abhänigkeit von der Dicke der $pO_2$ durchlässigen Membran flacher. Infolge dessen werden die Ansprechempfindlichkeit und der Rühreffekt geringer. Durch die Dicke der Membran können die entsprechenden Werte eingestellt bzw. reguliert werden.

Gleichwohl weist ein vorderer Bereich 17 gemäß Fig. 3b Nachteile auf, weil sich die Abschliff-Fläche infolge der Materialunterschiede als sehr inhomogen darstellt. Die Abschliff-Fläche weist von außen nach innen den Metallring der Metallrohrelektrode 14, den Ring aus Kunstharz 10, den Ring der Cellophanhülse 7,einen inneren Ring aus Kunstharz 10 und schließlich die Meßfläche 4 der Platindrahtelektrode 2 auf. Die bisher verwendeten Membnanen verbinden sich nicht hinreichend fest gleichzeitig mit allen Materialien. Zwar war bisher eine gute Haftung der Membran im Bereich des Kunstharzes gewährleistet, im Bereich des Metallringes der Metallrohrelektrode 14 stellten sich jedoch bei längerer Verwendung in Flüssigkeiten Abhebungserscheinungen ein. Dieses hatte zur Folge, daß die Elektrode nach jedem Gebrauch neu beschichtet werden mußte. Hinzu kam, daß erhebliche Änderungen der Meßwerte aufgrund der Drift auftraten.

Zwar ließ sich dieser Nachteil einer Elektrodenanordnung nach Fig. 3b durch eine Ausbildung des vorderen Bereiches 17 gemäß Fig. 3c verbessern, bei der die Abschliff-Fläche 23a linsenförmig hinter eine ebene Abschliff-Fläche 23 zurücktrat, jedoch entstand der Wunsch, die Ausbildung einer Elektrodenanordnung gemäß Fig. 3 zu verbessern.

Gemäß Fig. 3d wird hierzu eine sog. Recessionsstrecke 5 mit einer Länge e eingesetzt. Die Recessionsstrecke 5 wird elektrolytisch hergestellt. Zu diesem Zwecke wird zumindest der vordere Bereich 17 einer Elektrodenanordnung in eine Salzlösung gegeben und die Platindrahtelektrode 2 an den negativen Pol einer Gleichspannungsquelle und ein in die Salzlösung gelegtes Platinplättchen an den positiven Pol der Gleichspannungsquelle angeschlossen. Wenn nun ein Gleichstrom fließt, baut sich der vordere Bereich der Platindrahtelektrode 2 elektrolytisch ab. Der Vorgang ist zeit- und spannungsabhängig. In einem speziellen Fall wird als Salzlösung eine 3,5 Molare KCL-Lösung verwendet. Die Gleichspannung beträgt 5 Volt. Läßt man unter diesen Bedingungen für die Zeitdauer von 10 - 40 Sekunden einen Strom von 30 Milliampere fließen, so zieht sich das Platin der Platindrahtelektrode 2 um Bruchteile von mm hinter die Abschliff-Fläche 23 zurück.

Allein hierdurch kann der Meßstrom beim Einsatz der Elektrodenanordnung gegenüber einer blanken Elektrode nach Fig. 3a auf die Hälfte reduziert werden. Ebenso nimmt die Bewegungsempfindlichkeit bzw. Rührempfindlichkeit einer Elektrodenanordnung nach Fig. 3d merklich ab.

In einem bevorzugten Ausführungsbeispiel ist der vordere Bereich 17 einer Elektrodenanordnung gemäß Fig. 3 durch eine Membran 12, 12a beschichtet. Dieses Beschichten geschieht unter Vakuum. Der Teil 12a der Membran 12 füllt dabei die Recessionsstrecke 5 auf. Hierdurch läßt sich eine definierte Dicke der Membran 12, 12a erzielen. Hinzukommt, daß die Membran an der Kunstharzwand besser haftet, so daß Ablösungserscheinungen der Membran 12 nicht auftreten. Elektrodenanordnungen der Bauart nach Fig. 3e haben bei Tierversuchen bis zu über 48 Stunden im Gewebe gelegen und keinerlei Drift oder sonstige Änderungen der Meßwerte verursacht. Die Eichkurven von Elektrodenanordnungen gemäß Fig. 3e vor und nach dem Versuah änderten sich um weniger als 5%.

Fig. 4 zeigt im Querschnitt den Kunststoffkörper 26 und die Metallrohrelektrode 14 zusammen mit einer Einführhilfe 35, die ein Innenrohr 36 aufweist, in dessen Einführende 37 Schlitze 38 vorgesehen sind, in welche Fixierstifte 31 am Kunststoffkörper 26 hineinpassen. Durch das Innenrohr 36 sind die Leitungen 51 und 52 geführt, die aus seinem oberen Ende herausragen.

Der Kunststoffkörper 26 sitzt so leichtgängig im Einführende 37 des Innenrohres 36, daß dieses ohne Schwierigkeiten vom Kunststoffkörper 26 abgezogen werden kann, wenn die Metallrohrelektrode 14 in das Gewebe 50, beispielsweise in die Kopfhaut eines Kindes eingedreht worden ist.

Das Bedienungsende 39 des Innenrohres 36 weist einen Rand auf, der, wie in Fig. 4 dargestellt, in eine Arretierungskappe 41 eingefügt und dort befestigt ist.

Um das Innenrohr 36 herum ist koaxial und in Achsrichtung der konzentrischen Rohre verschiebbar ein Außenrohr 43 gestebkt, welches mittels einer Verrastung 42 und einer Rastöffnung 49 in der in Fig. 4 dargestellten Stellung mit der Arretierungskappe 41 in Wirkverbindung steht. Hierbei ist ein Abstand 48 zwischen einem Rand 47 am Bedienungsende 46 des Außenrohres 43 und einem Bodenring 40 in der Arretierungskappe 41 vorgesehen. Der gleiche Abstand 48 besteht zwischen der vorderen Fläche 29 des Kunststoffkörpers 26 und dem vorderen Rand 45 am Einführende 44 des Außenrohres 43. Wenn mit Hilfe einer handgriffähnlichen

Anordnung die Verrastung 42 angehoben wird, rastet die nicht positionierte Rastnase der Verrastung 42 aus der Rastöffnung 49 aus und die Arretierungskappe 41 kann um den Abstand 48 gemäß Fig. 4 nach unten geschoben werden. Dabei wird das Innenrohr 36 soweit vorgeschoben, daß aus dem Einführende 44 des Außenrohres 43 lediglich die Metallrohrelektrode 14 herausragt.

In dieser Position kann gemäß Fig. 5 durch leichte Drehung unter Druck die offene Spirale der Metallrohrelektrode 14 in den vorangehenden Teil bzw. das Gewebe 50 der Kopfhaut eingedreht werden. Sobald die offene Spirale in die Kopfhaut eingedreht ist, läßt sich die Einführhilfe 35 zurückziehen, so daß, wie dieses Fig. 7 erkennen läßt, die Leitungen 51 und 52 entweder an den CTG-Kabelanschlußblock 58 oder an die Leitung 52 angeschlossen werden können. Hierzu sind Ministecker vorgesehen.

Über die Elektrodenkabel 55 sind an das $pO_2$-Meßgerät 53 bzw. an das CTG-Meßgerät 57 Einmal-Elektroden 54 aus Silber/Silberchlorit angeschlossen. Über ein weiteres Elektrodenkabel 59 kann eine zusätzliche Einmal-Elektrode 54 an das Meßgerät 57 angeschlossen sein.

Mit der neuen Elektrodenanordnung und mit den in Fig. 7 erläuterten Meßgeräten 53 und 57 kann neben dem jeweiligen Sauerstoffpartialdruck $pO_2$ im Gewebe des Kindes auch ein peripheres, kardiales Aktionspotential zur Bestimmung der Herzfrequenz abgeleitet werden. Sollte diese unter einen vorgegebenen Wert absinken,und für eine lange Zeitspanne auf dem abgesunkenen Wert verharren, müssen z.B. die Beschleunigung der Geburt oder andere klinische Maßnahmen eingeleitet werden.

Unter Bezugnahme auf die Fig. 6a, 6b und 6c wird die Wirkungsweise der Elektrodenanordnung nach der Erfindung erläutert.

Fig. 6a zeigt stark schematisiert, daß das sauerstoffreiche arterielle Blut über dünne Gefäße zum venösen Schenkel abfließt und hierbei in der Endstrombahn, die nur aus einer Epithelschicht besteht, $pO_2$ ins Gewebe abgibt. Die erfindungsgemäße Elektrodenanordnung kann im wesentlichen drei Positionen einnehmen :

Pos. 1, in welcher ein $pO_2$-Wert sehr nahe am arteriellen Wert gemessen wird,

Pos. 2, der hier gemessene $pO_2$-Wert liegt zwischen dem arteriellen und dem venösen Wert und

Pos. 3, der hier gemessene Wert liegt sehr nahe am venösen $pO_2$-Wert.

Der mit der erfindungsgemäßen Elektrodenanordnung gemessene $pO_2$-Wert entspricht allein aufgrund der vorstehenden theoretischen Erläuterung immer einem Differenzwert zwischen dem arteriellen und dem venösen Wert. Da beim Einlegen der Elektrodenanordnung nicht bekannt ist, welche Position die Elektrodenanordnung bzw. die Meßfläche einnehmen wird, wird als Meßergebnis immer nur eine Aussage über das Verhalten der $pO_2$-Werte im Gewebe bei einer Kreislaufalteration angegeben. Diese Messung ist - wie die Versuchsergebnisse zeigen - wichtiger als die Messung eines $pO_2$-Absolutwertes im Gewebe selbst. Mit dem Messen des Differenzwertes kann die tatsächliche Sauerstoffversorgung des Gewebes ermittelt werden, wobei die Haut repräsentativ ist auch für alle inneren Organe. Hinzu kommt, daß bei Kreislaufalterationen, beispielsweise bei Volumenmangel, die in Fig. 6a schwarz dargestellte Endstrecke der Strombahn durch muskuläre Veränderungen der Wand den Blutfluß und damit die $pO_2$-Abgabe an das Gewebe ändern. Dies bedeutet, daß die erfindungsgemäß durchgeführte $pO_2$-Messung sowohl ein Kriterium für die periphere Organdurchblutung bzw. Hautdurchblutung als auch für deren $pO_2$-Versorgung ist. Der $pO_2$-Abfall bei den durchgeführten Messungen unter der Geburt im oberen Abschnitt der Fig. 6a stellt daher den kreislaufdynamischen Anteil dar, während der zweite Anteil die echte $pO_2$-Regenerationsphase aufzeigt. Der Abfall des $pO_2$-Gehaltes zwischen dem arteriellen und dem venösen Anteil des Gewebes ist in Fig. 6b dargestellt.

Fig. 6c zeigt im oberen Bereich schematisch den $pO_2$-Verlauf während einer kreislaufdynamischen Depressionsphase des Kindes, z.B. bei einer Nabelschnur-Kompression während einer Wehe. Während einer solchen Depressionsphase 1. sinkt $pO_2$ im Gewebe ab bis auf einen niedrigsten Wert. Mit dem Nachlassen der Wehe und mit dem Nachlassen der Nabelschnur-Kompression setzt eine Regenerationsphase II. ein, die in eine Gewebe-$pO_2$-Regenerationsphase III. übergeht, deren Verlauf im wesentlichen davon abhängt, wie sich die Nabelschnur-Kompression zurückbildet.

Im unteren Bereich der Fig. 6c ist der Verlauf des kindlichen Herzfrequenzmusters während der beschriebenen Phasen I., II., III. dargestellt.

## Patentansprüche

1. Anordnung mit einer Einstichelektrodenanordnung zur kontinuierlichen $pO_2$-Messung und Messung der fösalen Herzfrequenz (FHR) im lebenden und unruhig bewegten Gewebe, beispielsweise in der Haut des vorangehenden Teiles eines Kindes während der Geburt, dadurch gekennzeichnet, daß die Einstichelektrodenanordnung aus einer äußeren spiralförmigen Metallrohrelektrode (14), einem darin befindlichen spiralförmigen Rohr aus Isoliermaterial (7, 10) und einer darin angeordneten inneren spiralförmigen Platinelektrode (2) besteht, daß die vordere Endfläche der Einstichelektrodenanordnung eine Abschliff-Fläche (23) mit einem Neigungswinkel (α) von etwa 20° gegenüber einer durch den vorderen Bereich (17) der Metallrohrelektrode (14) sich erstreckenden Querschnittsfläche ist, und daß das Anschlußende (6) der Einstichelektrodenanordnung in einem zylindrischen Kunststoffkörper (26) angeordnet ist, der lösbare Fixiermittel (31) zur Aufnahme der Einstichelektrodenanordnung (1) im Einführende (37) eines Innenrohres (36) einer Einführhilfe (35) aufweist, wobei die Platindrahtelektrode (2) an ein $pO_2$-Meßgerät (53) und die Metallrohrelektrode (14) an ein zweites Meßgerät, z.B. an ein CTG-Meßgerät (57) geführt sind, und daß beide Meßgeräte (53, 57) unabhängige Bezugselektroden (54) aufweisen.

2. Anordnung nach Anspruch 1, dadurch gekennzeichnet, daß das spiralförmige Rohr aus Isoliermaterial, das die Platindrahtelektrode (2) innerhalb der Metallrohrelektrode (14) umgibt, eine Cellophanhülse (7) ist.

3. Anordnung nach Anspruch 2, dadurch gekennzeichnet, daß der Innenraum der Metallrohrelektrode (14) zusätzlich mit Kunstharz (10) ausgefüllt ist.

4. Anordnung nach Anspruch 3, dadurch gekennzeichnet, daß die Metallrohrelektrode (14) aus $V_2A$-Stahl besteht.

5. Anordnung nach einem der Ansprüche 1 - 4, dadurch gekennzeichnet, daß de zylindrische Kunststoffkörper (26) eine Höhe (H) von etwa 5 mm und einen Durchmesser (D) von etwa 4,5 mm aufweist.

6. Anordnung nach einem der Ansprüche 1 - 5, dadurch gekennzeichnet, daß die Metallrohrelektrode (14) mit dem Anschlußende (6) der Platindrahtelektrode (2) außermittig im Randbereich der vorderen Fläche (29) des Kunststoffkörpers (26) sitzt.

7. Anordnung nach einem der Ansprüche 1 - 6, dadurch gekennzeichnet, daß die Platindrahtelektrode (2) einen Durchmesser (d) von etwa 20 µm und die Cellophanhülse (7) einen Aussendurchmesser (a) von etwa 200 µm und einen Innendurchmesser (i) von etwa 180 µm aufweisen.

8. Anordnung nach einem der Ansprüche 1 - 7, dadurch gekennzeichnet, daß die Metallrohrelektrode (14) einen Außendurchmesser (ad) von etwa 450 µm und einen Innendurchmesser (id) von etwa 300 µm aufweist.

9. Anordnung nach einem der Ansprüche 1 - 8, dadurch gekennzeichnet, daß die Metallrohrelektrode (14) mit der Platindrahtelektrode (2) als (Teil)-Spiralwindung mit einer Steigung (S) von ca. 30° und mit einem Außendurchmesser (A) ausgebildet ist, welcher kleiner ist als der Durchmesser (D) des Kunststoffkörpers (26).

10. Anordnung mit einer Einstichelektrodenanordnung zur kontinuierlichen $pO_2$-Messung und Messung der fösalen Herzfrequenz (FHR) im lebenden und unruhig bewegten Gewebe, beispielsweise in der Haut des vorangehenden Teiles eines Kindes während der Geburt, dadurch gekennzeichnet, daß die Einstichelektrodenanordnung aus einer äußeren spiralförmigen Metallrohrelektrode (14), einem darin befindlichen spiralförmigen Rohr aus Isoliermaterial (7, 10) und einer darin angeordneten inneren spiralförmigen Platindrahtelektrode (2) besteht, daß die vordere Endfläche der Einstichelektrodenanordnung eine Abschfliff-Fläche (23) mit einem Neigungswinkel (α) von etwa 20° gegenüber einer durch den vorderen Bereich (17) der Metallrohrelektrode (14) sich erstreckenden Querschnittsfläche ist, daß die Meßfläche (4) der Platindrahtelektrode (2) um eine Recessionsstrecke (5) hinter der Abschliff-Fläche (23) zurückversetzt ist, und daß das Anschlußende (6) der Einstichelektrodenanordnung in einem zylindrischen Kunststoffkörper (26) angeordnet ist, der lösbare Fixiermittel (31) zur Aufnahme der Einstichelektrodenanordnung (1) im Einführende (27) eines Innenrohres (36) einer Einführhilfe (35) aufweist, wobei die Platindrahtelektrode (2) an ein $pO_2$-Meßgerät (53) und die Metallrohrelektrode (14) an ein zweites Meßgerät, z.B. an ein CTG-Meßgerät (57) geführt sind, und daß beide Meßgeräte (53, 57) unabhängige Bezugselektroden (54) aufweisen.

11. Anordnung nach Anspruch 10, dadurch gekennzeichnet, daß die Recessionsstrecke (5) eine Länge (e) von < 10 µm hat.

12. Anordnung nach einem der Ansprüche 10 und 11, dadurch gekennzeichnet, daß die Abschliff-Fläche (23) und die Meßfläche (4) mit einer elektrisch isolierenden, gasdurchlässigen Memban (12) überzogen sind.

13. Anordnung nach Anspruch 12, dadurch gekennzeichnet, daß der rückwärtige Teil (12a) der Membran (12) die Recessionsstrecke (5) ausfüllt.

14. Verfahren zum Herstellen einer Elektrodenanordnung, dadurch gekennzeichnet, daß eine Platindrahtelektrode und ein diese umgebendes, elektrisch isolievendes Rohr in einer äußeren Metallrohrelektrode, vorzugsweise aus V-A-Stahl, gebrachtwird, wonach diese Metallrohrelektrode spiralförmig gewendelt und in einem zylindrischen Kunststoffkörper angeordnet wird und dadurch, daß eine Recessionsstrecke im vorderen Bereich, beispielsweise nach den Ansprüchen 10 und 11, durch elektrolytischen Abbauen der Platindrahtelektrode (4) hergestellt wird.

15. Verfahren nach Anspruch 14, dadurch gekennzeichnet, daß zumindest der vordere Bereich der Elektrodenanordnung in eine Salzlösung eingegeben wird, in der sich ein Platin-Plättchen befindet, woraufhin für die Dauer einer der gewünschten Länge der Recessionsstrecke entsprechend einstellbaren Zeitspanne die Platindrahtelektrode (4) an den Minuspol und das Platin-Plättchen an den Pluspol einer Gleichspannungsquelle angeschlossen werden, und daß anschließend eine gründliche Säuberung des vorderen Bereiches der Elektrodenanordnung erfolgt.

16. Verfahren nach Anspruch 15, dadurch gekennzeichnet, daß als Salzlösung eine 3,5 Molare KCL-Lösung verwendet wird.

17. Verfahren nach einem der Anspüche 15 und 16, dadurch gekennzeichnet, daß bei einer Gleichspannung von 5 V für eine Zeitspanne von 10 - 40 sec. ein Strom von 30 Milliampere eingestellt wird.

18. Verfahren nach einem der Ansprüche 14 - 17, dadurch gekennzeichnet, daß die Beschichtung des vorderen Bereiches der Elektrodenanordnung mit einer Membran und das Auffüllen der Recessionsstrecke mit dem Material der Membran im Vakuum erfolgt.

## Claims

1. A system including an insertion-type electrode assembly for continuous $pO_2$ measuresments in living and unevenly moved tissue, for example, in the skin of the precedent part of a child during childbirth, characterized in that the insertion-type electrode assembly comprises an outer spiral metal tube electrode (14), a spiral-shaped tube of insulating material (7,10) contained therein, and an internal spiral platinum electrode (2) located therein, that the front-sided end face of the insertion-type electrode assembly is a ground-off face (23) with an angle of inclination ($\lambda$) of about 20° with respect to the cross-sectional face extending through the front-sided area (17) of the metal tube electrode (14), and that the connecting end (6) of the insertion-type electrode assembly is disposed in a cylindrical plastics body (26) exhibiting detachable fixing means (31) for the accommodation of the insertion-type electrode assembly (1) in the introducing end (37) of an internal tube (36) of an introducing aid (35), with the platinum wire electrode (2) being guided to a $pO_2$-meter (53) and the metal tube electrode (14) being guided to a second meter, e.g. a CTG-meter (57), and that both meters (53,57) exhibit independent reference electrodes (54).

2. A system according to claim 1, characterized in that the spiral tube of insulating material surrounding the platinum wire electrode (2) within the metal tube electrode (14), is a cellophane sleeve (7).

3. A system according to claim 2, characterized in that the interior of the metal tube electrode (14), in addition, is filled with synthetic resin (10).

4. A system according to claim 3, characterized in that the metal tube electrode (14) is made of $V_2A$ steel.

5. A system according to any one of claims 1 to 4, characterized in that the cylindrical plastics body (26) is of a height (H) of approximately 5 mm and exhibits a diameter (D) of about 4.5 mm.

6. A system according to any one of claims 1 to 5, characterized in that the metal tube electrode (14), with the connecting end (6) of the platinum wire electrode (2) is seated eccentrically in the edge region of the front-sided face (29) of the plastics body (26).

7. A system according to any one of the preceding claims 1 to 6, characterized in that the platinum wire electrode (2) is of a diameter (d) of about 20/um and the cellophane sleeve (7) is of an outer diameter (a) of about 200 /um and an internal diameter (i) of about 180 /um.

8. A system according to any one of claims 1 to 7, characterized in that the metal tube electrode (14) is of an outer diameter (ad) of about 450 /um and an internal diameter (id) of about 300 /um.

9. A system according to any one of claims 1 to 8, characterized in that the metal tube electrode (14) with the platinum wire electrode (2) is designed as a (partial) spiral winding with a pitch (S) of about 30° and an outer diameter (A) smaller than the diameter (D) of the plastic body (26).

10. A system including an insertion-type electrode assembly for continuous $pO_2$ measurements in the living and unevenly moved tissue, for example, in the skin of the precedent part of a child during childbirth, characterized in that the insertion-type electrode assembly comprises an outer spiral metal tube electrode (14), a spiral tube of insulating material (7,10) contained therein, and an internal spiral platinum wire electrode (2) located therein, that the front-sided end face of the insertion-type electrode assembly is a ground-off face (23) with an angle of inclination ($\alpha$) of about 20° with respect to a cross-sectional face extending through the front-sided area (17) of the metal tube electrode (14), that the measuring face (4) of the platinum wire electrode (2) is offset backwards by a recess distance (5) behind the ground-off face (23), and that the connecting end (6) of the insertion-type electrode assembly is disposed in a cylindrical plastics body (26) exhibiting detachable fixing means (31) for the accommodation of the insertion-type electrode assembly (1) in the introducing end (27) of an internal tube (36) of an introducing aid (35), with the platinum wire electrode (2) being guided to a $pO_2$ meter (53), and the metal tube electrode (14) being guided to a second meter, for example, a CTG-meter (57), and that both meters (53,57) exhibit independent reference electrodes (54).

11. A system according to claim 10, characterized in that the recess distance (5) is of a length (e) of <10 /um.

12. A system according to any one of claims 10 and 11, characterized in that the ground-off surface (23) and the measuring surface (4) are coated with an electrically insulating and gas-permeable diaphragm (12).

13. A system according to claim 12, characterized in that the rearward part (12a) of the diaphragm (12) fills the recessed distance (5).

14. A process for the manufacture of an electrode assembly composed of a platinum wire electrode and a metal tube electrode surrounding the former and being electrically insulated thereagainst, preferably made of V-A-steel, with a recessed distance in the front-sided area, of the type as set out in claims 10 and 11, characterized in that the front-sided area of the platinum wire electrode (4) is electrolytically degraded.

15. A process according to claim 14, characterized in that at least the front-sided area of the electrode assembly is dipped into a salt solution in which is contained a small platimum plate, whereafter the platinum wire electrode (4) is connected to the negative terminal and the platimum plate is connected to the positive terminal of a d.c. source for the duration of a period of time adjustable to correspond to the desired length of the recessed distance, and that, subsequently, the front portion of the electrode assembly is thoroughly cleaned.

16. A process according to claim 15, characterized in that a 3.5 mole KCL solution is used as the salt solution.

17. A process according to any one of claims 15 and 16, characterized in that a current of 30 milliamperes is set at a d.c. voltage of 5 V for a time span of 10-40 seconds.

18. A process according to any one of claims 14 to 17, characterized in that the coating of the front-sided area of the electrode assembly with a diagphragm and the filling of the recessed distance with the material of the diaphragm is performed in vacuum.

**Revendications**

1. L'appareil comportant un ensemble d'électrode à l'introduction pour la (les) mesure(s) de $pO_2$ continue(s) dans le tissu vivant et mu de manière non-tranquille, par exemple dans la peau de la partie précédente d'un enfant pendant la naissance, caractérisé en ce que l'ensemble d'électrode à l'introduction consiste d'une électrode externe à tube métallique sous forme spiralée, d'une tube (7,10) spiralé de matière isolante prévu dans la dernière, et d'une électrode interne (2) de platine prévue dans le dernier, et que la surface terminale antérieurs de l'ensemble d'électrode à l'introduction est une surface aiguisée (23) ayant an angle d'inclination ($\alpha$) d'environ 20° vis-à-vis d'une surface de section s'étendant à travers de la partie (17) antérieur de l'électrode (14) à tube métalllique, et que l'extrémité de connexion (6) de l'ensemble d'électrode à l'introduction est prévue dans un corps (26) plastique cylindrique comportant des moyens à fixer (31) détachables pour recueillir l'ensemble d'électrode (1) dans l'extrémité d'introduction (37) d'un tube intérieur (36) d'un moyens de secours d'introduction (35), l'électrode de fils platine (2) étant approchée à l'appareil (53) pour le $pO_2$ mesurage, et l'électrode de tube métallique (14) etant approchée à un seconde appareil mesureur, for exemple un CTG appareil mesureur (57), et que les deux appareils mesureurs (53,57) comportent des électrodes de référence (54) indépendantes.

2. L'appareil selon la revendication 1, caractérisé en ce que le tube spiralé de matière isolante entournant l'électrode de fils platine (2) dans l'électrode de tube métallique (14), est une douille cellophan (7).

3. L'appareil selon la revendication 2, caractérisé en ce que l'interieur de l'électrode de tube métallique (14), en outre, est rempli de résine synthétique (10)

4. L'appareil selon la revendication 3, caractérisé en ce que l'électrode de tube métallique (14) consiste d'acier $V_2$ A.

5. L'appareile selon l'une des revendications 1 à 4, caractérisé en ce que le corps (26) plastique cylindrique est d'une hauteur (H) d'environ 5 mm et d'un diamètre (D) d'environ 4.5 mmm.

6. L'appareil selon l'une des revendications 1 à 5, caractérisé en ce que l'électrode de tube métallique (14) avec l'extrémité de connexion (6) de l'électrode de fils platine (2) est assise eccentriquement dans la partie marginale de la surface (29) antérieur du corps plastique (26).

7. L'appareil selon l'une des revendications 1 à 6, caratérisé en ce que l'électrode de fils platine (2) est d'un diamètre (d) d'environ 20 /um, et la douille cellophan (7) est d'un diamètre extérieur (a) d'environ 200 /um et d'un diamètre intérieur (i) d'environ 180 /um.

8. L'appareil selon l'une des revendications 1-7, caractérisé en ce que l'électrode de tube métallique (14) a un diamètre extérieur (ad) d'environ 450 mm et un diamètre intérieur (id) d'environ 300 /um.

9. L'appareil selon l'une des revendications 1 à 8, caracctérisé en ce que l'électrode de tube métallique (14) avec l'électrode de fils platine (2) est sous forme d'une spire (partielle) ayant un pas (S) d'environ 30° et un diamètre extérieure (A) plus petit que le diamètre (D) du corps plastique (26).

10. L'appareil comportant un ensemble d'électrode à l'introduction pour la (les) $pO_2$ mesure(s) dans le tissu vivant et mu de manière non tranquille, par exemple dans la peau de la partie précédante d'un enfant pendant

la naissance, caractérisé en ce que l'emsemble d'électrode à l'introduction consiste d'une électrode (14) externe de tube métallique spiralé, d' un tube spiralé (7,10) de matière isolante prévu dans la dernière et d'une electrode (2) interne de fils platine prévu dans le dernière, que la surface terminale antérieur de l'ensemble d'électrode à l'introduction est une surface aiguisée (23) ayant an angle d'inclination ($\alpha$) d'environ 20° vis-à-vis une surface de section s'étendant à travers de la partie (17) antérieure de l'électrode de tube métallique (14), que la surface de mesure (4) de l'électrode de fils platine (2) est offset sur le derrière par un intervalle (5) d'évidement derrière la surface aiguisée (23), et que l'extrémité de connexion (6) de l'ensemble d'électrode à l'introduction est prévue dans un corps plastique (26) cylindrique comportant des moyens à fixer detachables (31) pour receuillir l'ensemble d'électrode à l'introduction (1) dans l'extrémité d'introduction (27) d'un tube intérieur (36) d'un moyens de secour d'introduction (35), l'électrode de fils platine (2) etant approchée à un $pO_2$ instrument mesureur (53) et l'electrode de tube métallique (14) etant approchée à un second instrument mesureur, for example, un CTG mesureur (57), et que les deux mesureurs (53,57) comportent des électrodes à référence indépendantes.

11. L'appareil selon la revendication 10, caractérisé en ce que l'intervalle (5) d'évidement a une longueur (e) de > 10 /um.

12. L'appareil selon l'une des revendications 10 et 11, caractérisé en ce que la surface aiguisée (23) et la surface de mesure (4) sont couvertes d'une membrane (12) électriquement isolante et perméable à gaz.

13. L'appareil selon la revendication 12, caractérisé en ce que la partie (12a) à l'arrière de la membrane (12), remplit l'intervalle d'évidement (5).

14. Procéde de fabrication d'un ensemble d'électrode, composé d'une électrode de fils platine et et d'une électrode de tube métallique, préférablement d'acier V-A, entournant la dernière et étant isolée électriquement vers la méme, avec un intervalle d'évidement dans la partie antérieure, par exemple selon les revendications 10 et 11, caractérisé en ce que la partie antérieure de l'électrode de fils platine (4) est dégradée électrolytiquement.

15. Procédé selon la revendication 14, caractérisé en ce qu'au moins la partie antérieure de l'ensemble d'électrodes est introduite dans une solution saline dans laquelle se trouve une plaque platine, après quoi l'électrode de fils platine (4) pour la durée d'un laps de temps ajustable correspondant à la longueur désirée de l'intervalle d'évidement, est raccordée au pol négatif et la plaque platine est raccordée au pol positif d'dune source de c.c. voltage, et qu'après cela la partie antérieur de l'ensemble d'électrodes est nettoyée profondément.

16. Procédé selon la revendication 15, caractérisé en ce qu' une KCL-solution aux 35 molaires est employée comme solution saline.

17. Procédé selon l'une des revendications 15 et 16, caractérisé en ce qu' un courant au 30 milliamperes est ajusté à un c.c. voltage au 5 V pour un laps de temps de 10-40 secondes.

18. Procédé selon l'une des revendications 14 à 17, caractérisé en ce que la couverture de la partie antérieure de l'ensemble d'électrodes avec une membrane et le remplissage de l'intervalle d'évidement avec la matière de la membrane sont fait dans le vide.

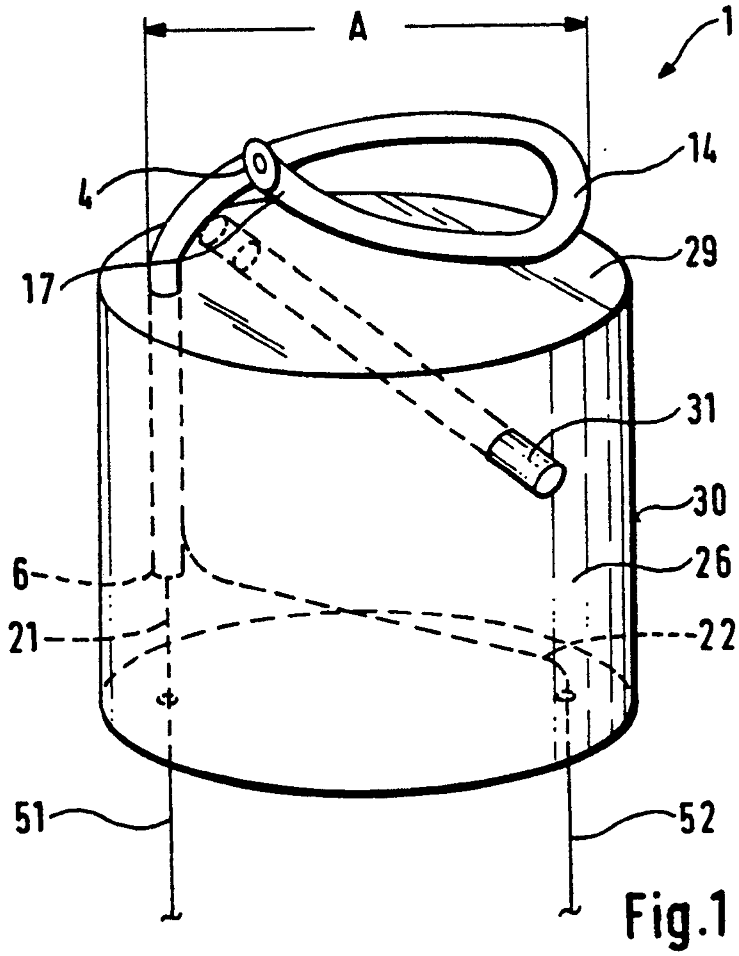


Fig.1

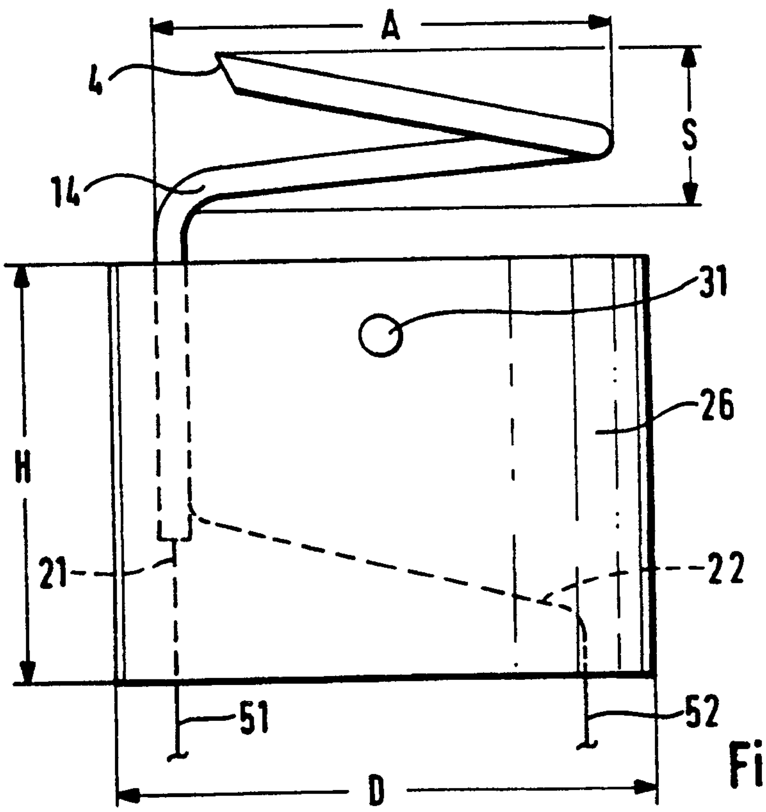


Fig.2

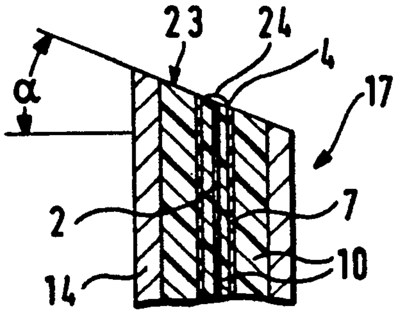


Fig.3a

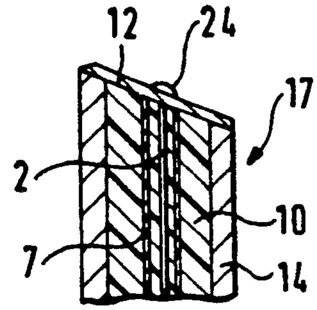


Fig.3b

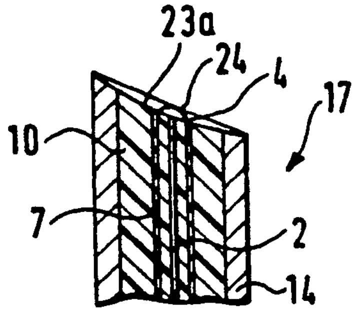


Fig.3c

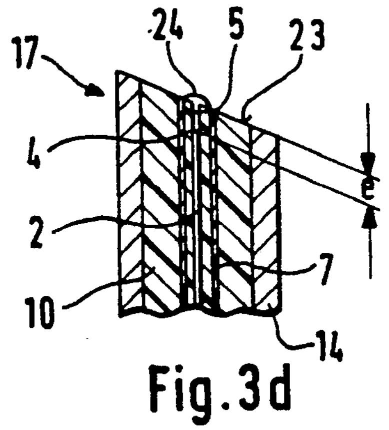


Fig.3d

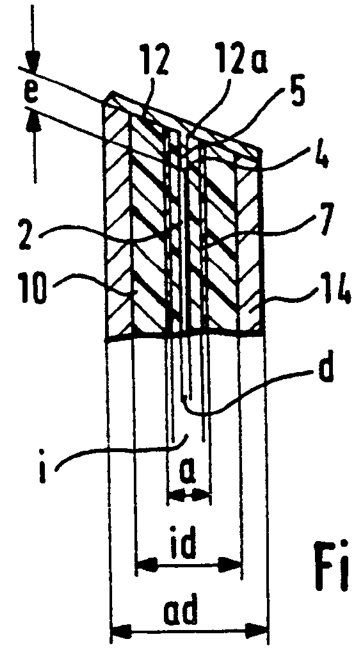


Fig.3e

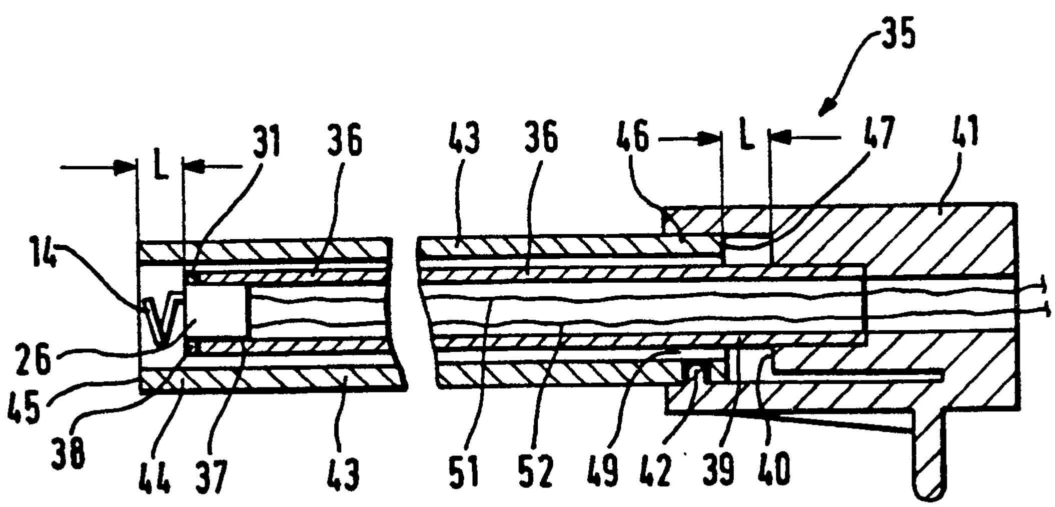


Fig.4

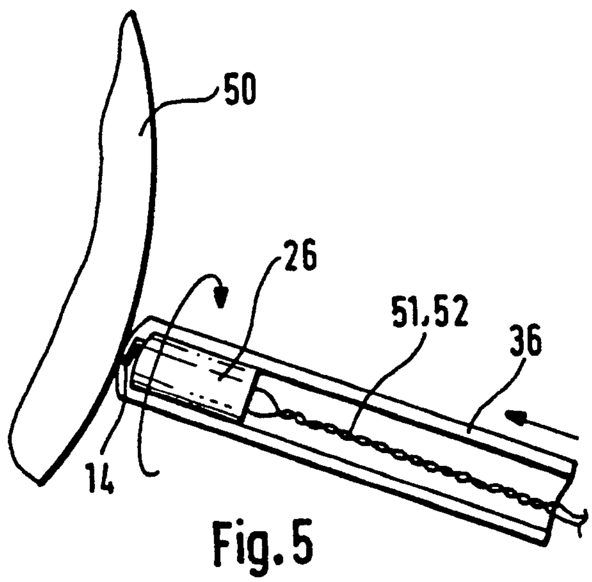


Fig.5

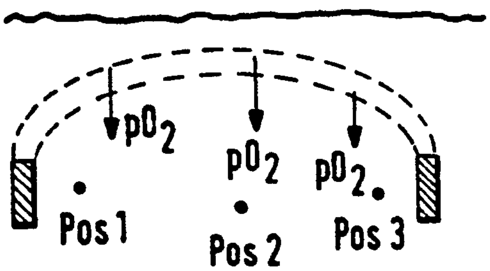


Fig.6a

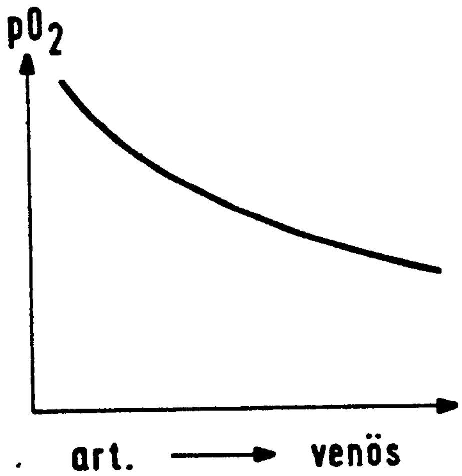


Fig.6b

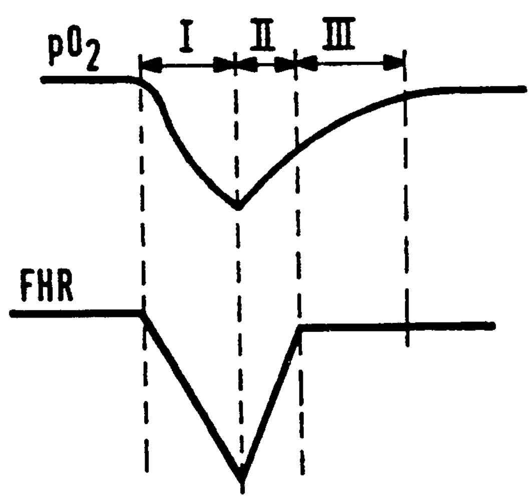


Fig.6c

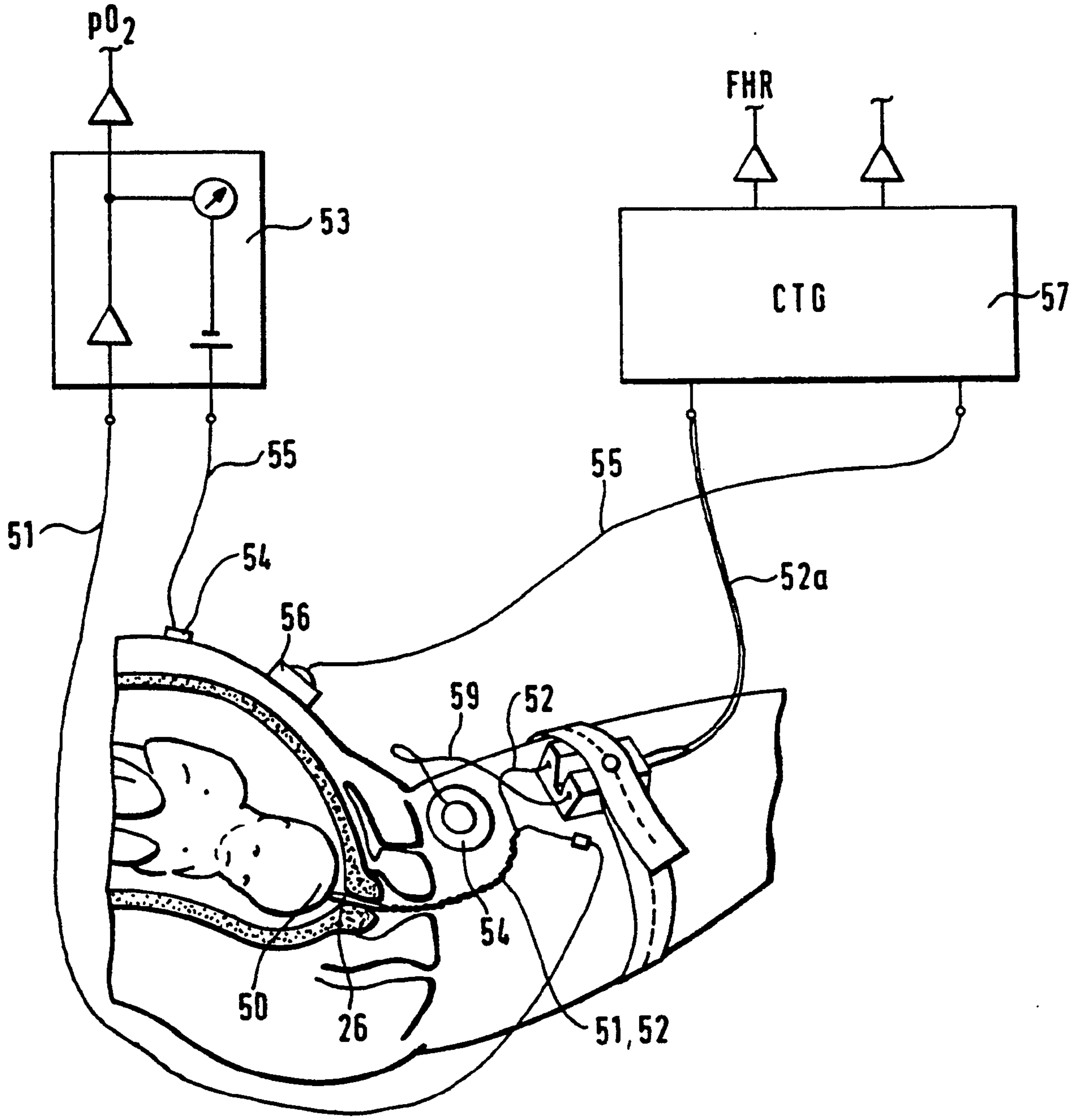


Fig.7